# EUROPEAN PATENT SPECIFICATION

(11) **EP 3 677 670 B1**
(45) Date of publication and mention of the grant of the patent: **19.06.2024**
(21) Application number: 18851336.0
(22) Date of filing: 27.08.2018
(51) Int. Cl.: C12N 5/0775, C12N 5/071

(54) **CELL SHEET HAVING FIBROSIS INHIBITORY ACTION**
ZELLBLATT MIT FIBROSE-INHIBIERENDER WIRKUNG
FEUILLE CELLULAIRE PRÉSENTANT UNE ACTION INHIBITRICE DE LA FIBROSE

(30) Priority: 01.09.2017 JP 2017168118
(43) Date of publication of application: 08.07.2020
(73) Proprietor: National University Corporation Tottori University, Tottori-shi, Tottori 680-8550 (JP); KanonCure, Inc., Yonago-shi, Tottori 683-0826 (JP)
(72) Inventor: SHIOTA , Goshi, Yonago-shi Tottori 683-8503 (JP); ITABA, Noriko, Yonago-shi Tottori 6838503 (JP); KONO, Yohei, Yonago-shi Tottori 683-0826 (JP)
(74) Representative: Gulde & Partner
(86) International application number: PCT/JP2018/031537
(87) International publication number: WO 2019/044753

(56) References cited:
- EP-A1- 2 698 365
- WO-A1-2015/147107
- WO-A1-2017/047762
- WO-A1-2019/235362
- US-A1- 2017 216 362
- ITABA ET AL: "P7-1 Hepatic fibrosis inhibitory action by bone marrow-derived mesenchymal stem cell hepatocyte sheet", PROGRAMS AND ABSTRACTS OF THE 23RD JAPANESE SOCIETY FOR THE RESEARCH OF HEPATIC CELLS; JULY 7-8, 2016, JAPANESE SOCIETY FOR THE RESEARCH OF HEPATIC CELLS, JAPAN, vol. 23, 30 June 2016 (2016-06-30), page 65, XP009519168,
- KOUNO ET AL: "P4-5 Hepatic fibrosis inhibitory effect of human mesenchymal stem cell hepatocyte sheet transplantation", PROGRAMS AND ABSTRACTS OF THE 22ND JAPANESE SOCIETY FOR THE RESEARCH OF HEPATIC CELLS; JUNE 4-5, 2015, JAPANESE SOCIETY FOR THE RESEARCH OF HEPATIC CELLS, JAPAN, vol. 22, 31 May 2015 (2015-05-31), page 63, XP009519167,
- ITABA ET AL: "O-424 Hepatic fibrosis inhibitory effect of mesenchymal stem cell-derived hepatocyte sheet", KANO-ACTA HEPATOLOGICA JAPAN, NIHON KANZO KYOKAI, TOKYO, JP, vol. 58, no. Suppl. 1, 20 April 2017 (2017-04-20), pages A411 #O-424, XP009518992, ISSN: 0451-4203
- NORIKO ITABA ET AL: "Human mesenchymal stem cell-engineered hepatic cell sheets accelerate liver regeneration in mice", SCIENTIFIC REPORTS, vol. 5, no. 1, 10 November 2015 (2015-11-10), XP055759964, DOI: 10.1038/srep16169
- Itaba, Noriko et al.: "P7-1 Hepatic fibrosis inhibitory action by bone marrow-derived mesenchymal stem cell hepatocyte sheet", Programs and Abstracts of the 23rd Japanese Society for the Research of Hepatic Cells; July 7-8, 2016, vol. 23, July 2016 (2016-07), page 65, XP009519168, JAPAN
- Kouno, Youhei et al.: "P4-5 Hepatic fibrosis inhibitory effect of human mesenchymal stem cell hepatocyte sheet transplantation", Programs and Abstracts of the 22nd Japanese Society for the Research of Hepatic Cells; June 4-5, 2015, vol. 22, June 2015 (2015-06), page 63, XP009519167, JAPAN
- Itaba, Noriko et al.: "Hepatic fibrosis inhibitory effect of mesenchymal stem cell-derived hepatocyte sheet (non-official translation)", Kanzo = Acta hepatologica Japonica, vol. 58, no. suppl. 1, #O-424, 20 April 2017 (2017-04-20), page A411, XP009518992, Japan
- Noriko Itaba, Yoshiaki Matsumi, Kaori Okinaka, An Afida Ashla, Yohei Kono, Mitsuhiko Osaki, Minoru Morimoto, Naoyuki Sugiyama, Kaz: "Human mesenchymal stem cell-engineered hepatic cell sheets accelerate liver regeneration in mice", Scientific Reports, vol. 5, 16169, 10 November 2015 (2015-11-10), pages 1-17, XP009509936, DOI: 10.1038/srep16169
- Itaba, Noriko et al.: "O-41-5 Hepatic fibrosis inhibitory action of human mesenchymal stem cell hepatocyte sheet", Regenerative Medicine, vol. 16, 1 February 2017 (2017-02-01), pages 1-3, XP009519165, ISSN: 1347-7919
- Kouno, Youhei et al.: "O6-2 Effect of liver disease therapeutic cell sheet on repairing tissue with respect to hepatic fibrosis pathology", Programs and Abstracts of the 24th Japanese Society for the Research of Hepatic Cells, vol. 24, 7 August 2017 (2017-08-07), pages 1-2, XP009519164, Japan
- Kouno; Youhei: "O-169 Review on anti-fibrosis action by human mesenchymal stem cell hepatocyte sheet and mechanism of anti-fibrosis action", Kano-ACTA Hepatologica Japan, vol. 57, no. S1, 1 January 2016 (2016-01-01), pages 1-1, XP009518993, JP
- Kouno, Youhei et al.: "O-29-5 Therapeutic effect of mesenchymal stem cell hepatocyte sheet on hepatic fibrosis model, and action mechnism", Regenerative Medicine, vol. 15, #O-29-5, 2016, page 242, XP009519166, JAPAN ISSN: 1347-7919
- Shiota, G.; Itaba, N.: "Progress in stem cell-based therapy for liver disease", Hepatology Research, vol. 47, no. 2, 17 June 2016 (2016-06-17), pages 127-141, XP055669627, DOI: 10.1111/hepr.12747
- Akimoto, J. et al.: "Facile cell sheet manipulation and transplantation by using in situ gelation method", Journal of Biomedical Materials Research part B: Applied Biomaterials, vol. 102, no. 8, 24 March 2014 (2014-03-24), pages 1659-1668, XP055669630, DOI: 10.1002/jbm.b.33148

## Description

### TECHNICAL FIELD

The present disclosure relates to a cell sheet to be used in regenerative medicine. More specifically, the present disclosure relates to a cell sheet having enhanced fibrosis inhibitory activity.

### BACKGROUND ART

"Regenerative medicine" makes possible treatment to restore not only the function but also the structure of the body to its original condition, or as close as possible thereto, by utilizing cells that have been increased in number by culture, etc. To increase the number of cells, there is a method that injects the cells themselves. However, in order to make the repair function possessed by the cells work in a predetermined tissue, the transplanted cells must be integrated into the tissue and fixed there.

A cell sheet is obtained by culturing cells collected from a patient's tissue or an established cell line and making them into a sheet. A certain amount of cells that have been increased by culture are bonded to each other to form a single-layer sheet. Since adhesion proteins such as the extracellular matrix are maintained at the bottom of the cell sheet, the cell sheet engrafts to the transplant tissue without surgical suturing. The engrafted cell sheet releases humoral factors and promotes regenerative healing. Human transplantation and regenerative medicine have been performed in the cornea, heart, esophagus, etc. to date, and the range of applications is expected to expand in the future. The preparation of a cell sheet from mesenchymal stem cells has been described in the prior art, e.g. in EP 2 698 365 A1, US 2017/216362 A1 and by Noriko Itaba et al in "Human mesenchymal stem cell-engineered hepatic cell sheets accelerate liver regeneration in mice", SCIENTIFIC REPORTS, 2015, vol. 5, no. 1, XP055759964.

The present inventors reported differentiating mesenchymal stem cells and patient bone marrow-derived mesenchymal stem cells into cells having liver function using a compound having a Wnt/β-catenin signal inhibitory action, making the differentiated cells into a sheet, stacking a plurality of the sheets, and suppressing liver damage in mice (Patent Reference 1).

Tissue fibrosis is a symptom of liver damage. Upon progressing in the liver, fibrosis becomes cirrhosis and liver cancer. Fibrosis also occurs in the lung, kidney, heart, skin, etc. Non-Patent Reference 1 describes the results of a clinical trial of pirfenidone as relates to the treatment of fibrosis.

### PRIOR ART REFERENCES

### PATENT REFERENCES

Patent Reference 1: WO2012/141038 A1

### NON-PATENT REFERENCES

Non-Patent Reference 1: Nobel et al., Lancet, May 21; 377(9779): 1760-9

### SUMMARY OF THE INVENTION

### PROBLEMS TO BE SOLVED BY THE INVENTION

While research results relating to fibrosis treatment are gradually accumulating, there are still few effective therapeutics for treatment of fibrosis, and side effects may also be a problem for some patients in treatment by compounds such as the above. Therefore, conventional fibrosis inhibitors alone are not adequate, and it is necessary to establish therapeutic methods in regenerative medicine where side effects do not pose a problem, especially therapeutic methods using a cell sheet.

In order to obtain a predetermined therapeutic effect in humans using the present inventors' abovementioned cell sheet, it is estimated that a stack of two or three cell sheets obtained by culturing in a culture dish 15 cm in diameter must be applied to multiple locations.

In addition, liver cells differentiated from mesenchymal stem cells are used in the above reference. It is difficult to strictly manage and comprehend the number of differentiated cells in all samples in differentiated cell sheets; therefore, managing cell sheets such as the above as a product is labor-intensive.

Given the above circumstances, the cost per target specimen tends to rise in regenerative medicine, which has hindered the realization and spread of regenerative medicine products, etc.

### MEANS USED TO SOLVE THE ABOVE-MENTIONED PROBLEMS

The present invention is directed to subject matter as defined in the appended claims.
As a result of in-depth studies conducted to solve problems such as the above, the present inventors discovered a means that makes it possible to enhance the fibrosis inhibitory action in a cell sheet comprising bone marrow mononuclear cells.

This method also shows a similar tendency when bone marrow mononuclear cells are treated by a compound having a Wnt/β-catenin signal inhibitory effect, and the fibrosis inhibitory effect was discovered to be even stronger in this case, leading to the present invention.

Specifically, according to one embodiment of the present disclosure:
there is provided a cell sheet comprising bone marrow mononuclear cells, wherein the cell sheet is obtained by making bone marrow mononuclear cells from a suspension of bone marrow mononuclear cells into a sheet, shrinking the sheet, and then suspension culturing the sheet for at least 8 hours.

In this cell sheet, the bone marrow mononuclear cells may be adherent bone marrow mononuclear cells.

In this cell sheet, the bone marrow mononuclear cells may be mesenchymal stem cells.

In this cell sheet, the bone marrow mononuclear cells may be exposed to a compound having a Wnt/β-catenin signal inhibitory action when being made into a sheet, and this compound may be IC-2.

This cell sheet has a high fibrosis inhibitory effect and is applicable to various tissues.

This cell sheet has high MMP activity and is useful as a cell sheet for treating liver disease.

Also provided in the present disclosure is a laminated cell sheet for treating fibrosis obtained by laminating the aforedescribed cell sheets.

Also provided in the present disclosure is an in vitro method for producing a cell sheet having a fibrosis inhibitory action. This method includes a step for obtaining bone marrow mononuclear cells, a step for suspending the bone marrow mononuclear cells in culture broth, a step for seeding the suspension of bone marrow mononuclear cells onto a temperature-responsive culture dish and incubating, a step for confirming that the cultured bone marrow mononuclear cells have become confluent, moving the temperature-responsive culture dish into an environment of 32°C or lower, and obtaining a cell sheet, and a step for suspension culturing the cell sheet obtained for at least 8 hours.

In this method, the bone marrow mononuclear cells may be adherent bone marrow mononuclear cells or mesenchymal stem cells. Also, the culture broth may contain a compound having a Wnt/β-catenin signal inhibitory action in the step in this method for seeding the suspension of bone marrow mononuclear cells onto a temperature-responsive culture dish and incubating.

In this method, the compound having a Wnt/β-catenin signal inhibitory action may be IC-2.

In this method, the cell sheet obtained is applicable to various tissues.

The cell sheet obtained in this method is useful as a cell sheet for treating liver disease.

While not intending to be bound by theory, it is thought that the cell sheet of the present disclosure maintains a single-layer confluent state and, by being shrunk, the gap junctions between cell membranes are strengthened and, by being suspension cultured for a certain time, the intercellular activity is enhanced by causing small molecular metabolites, ions, etc. to be shared between the cells that constitute the cell sheet.

Degeneration and deterioration of a cell sheet pose a problem during suspension culture. For example, when a cell sheet for treating ischemic heart disease comprising skeletal myoblasts is stored at from 15°C to 25°C after preparation, the cell sheet must be applied to the patient within 10 hours. The present inventors confirmed that when a cell sheet is produced from bone marrow mononuclear cells as described in the present disclosure, the cell sheet presents high MMP activity for at least 8 hours or up to 24 hours after production, even when allowed to stand in a 20°C, 5% CO₂ environment.

### ADVANTAGES OF THE INVENTION

A cell sheet having an enhanced fibrosis inhibitory effect in the cell sheet is obtained in accordance with the present disclosure.

### BRIEF DESCRIPTION OF THE DRAWINGS

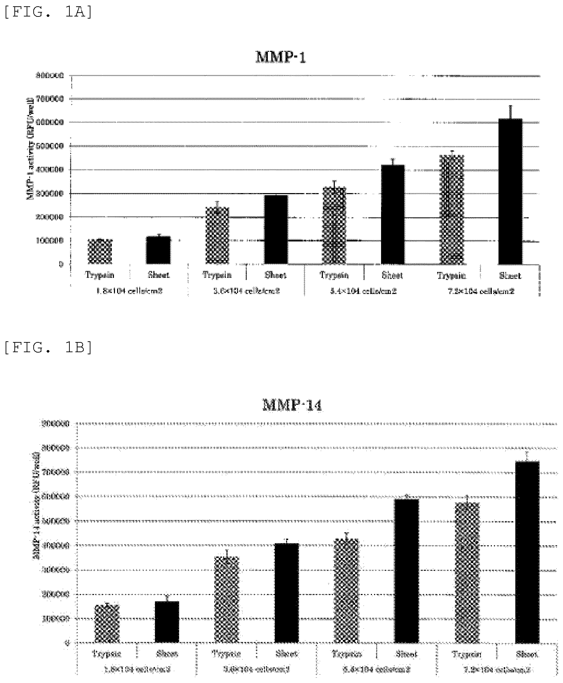

(FIG. 1A) The results in FIG. 1A show the results on MMP-1 per cell (FIG. 1A). It was revealed that, when cultured at increased seeding density, MMP-1 was higher in cell sheets subjected to a suspension culturing after making the sheet than in a trypsin-treated cell suspension. Also, it was revealed that the MMP-1 activity per cell increased when the cell sheet of the present disclosure was made by increasing the cell seeding density. It was a surprising result that the MMP-1 activity per cell was increased by increasing the seeding density. In addition, while the activity increased about four-fold in the trypsin-treated cell suspension when the seeding density was increased four-fold, the activity increased about six-fold in the cell sheet of the present disclosure. Also, cell sheets of high cell seeding density also had excellent physical strength.

(FIG. 1B) It was revealed that, when cultured at increased seeding density, MMP-14 was higher in cell sheets subjected to a suspension culturing after making the sheet than in a trypsin-treated cell suspension. Also, it was revealed that the MMP-14 activity per cell increased when the cell sheet of the present disclosure was made by increasing the cell seeding density. It was a surprising result that the MMP-14 activity per cell was increased by increasing the seeding density. In addition, while the activity increased about four-fold in the trypsin-treated cell suspension when the seeding density was increased four-fold, the activity increased about five-fold in the cell sheet of the present disclosure. Also, cell sheets of high cell seeding density also had excellent physical strength.

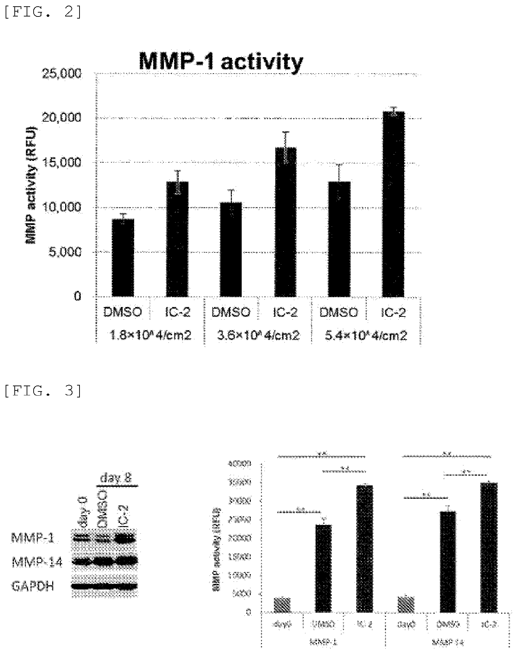

(FIG. 2) FIG. 2 shows the results on MMP-1 in a cell sheet when the suspension culture step was carried out overnight (eight hours) using adherent bone marrow mononuclear cells. Suspension culturing overnight increased the MMP-1 activity in DMSO treatment and in IC-2 treatment. Although not shown in the drawing, the results were also the same for MMP-14. Also, in addition to Example 1, it was demonstrated that the activity relative to protein is also increased by increasing the seeding density. In addition, this tendency was more significant in cell sheets treated by IC-2 than by DMSO.

(FIG. 3) FIG. 3 shows that the MMP-1 and MMP-14 activity increased in a cell sheet obtained from bone marrow-derived mesenchymal stem cells according to the method of the present disclosure. Day 0 is the MMP-1 and MMP-14 activity measured in samples prior to IC-2 and DMSO treatment. The cells at this time are not in the form of a sheet. The drawing shows that these activities increased further when DMSO and IC-2 were used in the sheetmaking step and that the MMP-1 and MMP-14 activity was higher in cell sheets treated by IC-2 than by DMSO.

### BEST MODE FOR CARRYING OUT THE INVENTION

### <Bone marrow mononuclear cells>

The cell sheet of the present disclosure can be produced from bone marrow mononuclear cells. Bone marrow contains hematopoietic and mesenchymal stem cells that can be differentiated into vascular endothelial cells, myocardial cells, smooth muscle cells, etc.

When bone marrow cells are cultured under specific conditions, a stromal layer of adherent cells adheres to the substrate and proliferates. The stromal layer is constituted from a variety of cell types such as fibroblasts, mesenchymal stem cells, adipocytes, endothelial cells, macrophages, etc. In the present disclosure, bone marrow mononuclear cells means a cell group in a fraction separated from bone marrow aspirate by density gradient centrifugation. By subculturing bone marrow mononuclear cells, it is also possible to increase the cells in the stromal layer and produce a cell sheet.

Bone marrow mononuclear cells can be collected from human or animal bone marrow and can also be obtained from Lonza Japan Ltd., etc.

### <Cell sheet>

One embodiment of the present disclosure is a cell sheet comprising bone marrow mononuclear cells produced as described above. This cell sheet has high fibrosis inhibitory activity, as explained later in the examples.

Also, one embodiment of the present disclosure relates additionally to a cell sheet obtained by treating a cell sheet comprising bone marrow mononuclear cells by a compound having a Wnt/β-catenin signal inhibitory action. This sheet is characterized by further increased fibrosis inhibitory activity.

This cell sheet may be used for liver surface transplantation but can be applied to inhibit fibrosis of any tissue as long as there is no rejection reaction.

When this cell sheet is transplanted, one or multiple layers may be transplanted. Also, the transplant location may be one site or multiple sites. Furthermore, if multiple, the number may be, for example, 2, 3, 4, 5, or 6, or more, or within this range.

The cell sheet in the present disclosure has a higher fibrosis inhibitory effect than in the prior art. The fibrosis inhibitory effect can be assessed by the fact that the matrix metalloprotease (MMP) activity is increased and by the hydroxyproline level and histological findings. Regarding the advance of fibrosis in living tissue, it has been revealed that activated fibroblasts (hepatic stellate cells in the liver) accumulate at the fibrotic site and produce large amounts of type I collagen. The disclosed cell sheet increases the activity of MMP-1 and MMP-14 which decompose this type I collagen.

Also, in one aspect of the present disclosure, the cell sheet is a cell sheet comprising bone marrow mononuclear cells. As along as no rejection reaction occurs, this sheet can be used to inhibit fibrosis in various tissues.

### <Production of cell sheet>

Bone marrow mononuclear cells are seeded onto a temperature-responsive reaction dish containing medium. An example of the medium is DMEM/20% FBS containing bFGF. The number of cells seeded is, for example, 1.3 × 10⁴ cells/cm². The number can be modified as is appropriate in accordance with the flask size and culture environment and is within the range of knowledge of those skilled in the art. While changing the medium every four days, the cells are cultured at 37°C, 5% CO₂ until confluent.

In the present disclosure, confluent means a state in which the seeded cells are in close contact on the Petri dish surface without any gaps. In the present disclosure, the percentage of confluency is not important, but a state in which from 70% to 90% of the Petri dish surface is visibly densely covered by cells is taken to be confluent. Furthermore, the cells become confluent in about eight days when 1.3 × 10⁴ cells/cm² cells are cultured at 37°C, 5% CO₂ in a Petri dish 6 cm in diameter. The medium is changed every four days, as a general rule.

The cells can be made into a sheet by moving the culture Petri dish that has become confluent into a 5% CO₂ incubator kept at 32°C or lower. "Made into a sheet" means that, when the cell sheet cultured for eight days in a temperature-responsive culture dish at 37°C, 5% CO₂ is moved into a 5% CO₂ incubator kept at 32°C or lower, the cells are detached from the cell adhesion surface of the temperature-responsive culture dish due to change of nature of surface of the dish from hydrophobic to hydrophilic, thereby cells of a sheet-like structure can be recovered. Since adherent proteins such as the extracellular matrix, etc. are maintained at the bottom of this cell sheet, the recovered cell sheet can be engrafted to tissue.

In the present disclosure, "kept at 32°C or lower" means that the incubator is set so as not to exceed 32°C. The temperature when a sheet is made varies depending on the seeded cells; 32°C is acceptable when making a sheet from adherent bone marrow mononuclear cells or mesenchymal stem cells, but 20°C is preferred in the sense of avoiding high temperature as much as possible since making a sheet takes time when the cells have been treated with a compound having a Wnt/β-catenin signal inhibitory action.

In the step that produces a cell sheet of the present disclosure, the cells made into a sheet are shrunk. For example, a cell sheet made in a temperature-responsive culture dish 6 cm in diameter becomes about 1 cm in diameter. Shrinkage makes it easier to adjust the number of cells applied to the affected site. In the present disclosure, the proportion of shrinkage is not important, and there is no problem as long as the difference in size is not great enough to interfere with lamination when a plurality of the cell sheets are stacked, which is one aspect of the present disclosure.

One aspect of the present disclosure is to control the seeding density to increase the MMP activity per cell in the cell sheet. In plate culture, the number of cells seeded is selected as is appropriate depending on the ease of increasing the cells seeded and the culture conditions such as the container. For example, the number of cells seeded to a 6 cm culture dish is selected to be 9.0 × 10³ cells/cm² when obtaining a liver cell sheet by differentiating mesenchymal stem cells (Patent Reference 1). In a cell sheet for regenerative medicine according to the present disclosure, it is important to obtain accurate single-layer sheets, which are then laminated as is appropriate and used in treatment. Seeding more than the well-proven number of seeded cells was thought to pose a risk from the viewpoint of obtaining an accurate single-layer sheet. The present inventors, however, discovered that the MMP activity increases markedly in cell sheets with an increased number of cells seeded.

In another aspect of the present disclosure, bone marrow mononuclear cells are exposed to a compound having a Wnt/β-catenin signal inhibitory effect during culture in the temperature-responsive culture dish. In this case, the medium is changed for a medium (DMEM/10% FBS) containing a predetermined amount of a compound having a Wnt/β-catenin signal inhibitory effect on the next day of seeding. As one example, a concentration of 30 µM is preferred if the compound is IC-2 as will be described later. After refreshing the medium with a medium containing a compound having a Wnt/β-catenin signal inhibitory effect in the same manner as above on 5 days after seeding, then the medium is changed for a medium free of Wnt/β-catenin signal inhibitory compound on 8 days after seeding, and the culture is moved to a 5% CO₂ incubator kept at 32°C or lower to make a sheet.

In another aspect of the present disclosure, bone marrow mononuclear cells can also be propagated as an adherent cell population prior to making a sheet in the temperature-responsive culture dish. As an example of obtaining this population, after conducting plate culture until confluent in medium (DMEM/20% FBS) containing 20 ng/mL of bFGF, the Petri dish is washed with PBS, the cells are detached from the culture dish by trypsin and recovered, and the cell fraction can be obtained by removing the supernatant by centrifugation. The fraction is suspended in medium in the same way as the bone marrow mononuclear cells, and the cells are seeded onto a new culture dish. The medium is changed for a medium of the same composition every four days after seeding. When confluent, the cells are washed with PBS, the cells are detached from the culture dish by trypsin and recovered, and a predetermined number of cells are seeded onto a temperature-responsive culture dish to obtain a cell sheet. If necessary, the adherent cell fraction may be subcultured as is appropriate prior to making a sheet in the temperature-responsive culture dish, but a suitable range in the present disclosure is up to three passages.

In addition, in another aspect of the present disclosure, the subcultured adherent cells may be exposed to a Wnt/β-catenin signal inhibitory compound during culture in a temperature-responsive culture dish. In this case, the medium is changed for a medium (DMEM/10% FBS) containing a predetermined amount of a compound having a Wnt/β-catenin signal inhibitory effect on the next day of seeding. As one example, a concentration of 30 µM is preferred if the compound is IC-2 as will be described later. After refreshing the medium with a medium containing a compound having a Wnt/β-catenin signal inhibitory effect in the same manner as above on 5 days after seeding, then the medium is changed for a medium free of Wnt/β-catenin signal inhibitory compound on 8 days after seeding, and the culture is moved to a 5% CO₂ incubator kept at 32°C or lower to make a sheet.

### <Wnt/β-catenin signal inhibitory compound>

In the present disclosure, Wnt/β-catenin signal inhibitory compounds include compounds described in Japanese Patent Application Publication No. 2011-219435, WO2012/141038 A1, WO2015/147107 A1, and WO2017/047762 A1.

Suitable compounds are the IC-2 described in WO2012/141038 A1 and derivatives thereof. Suitable compounds are one or more compounds selected from the compound groups shown by formula (1) and formula (2), salts thereof, and solvates of these.

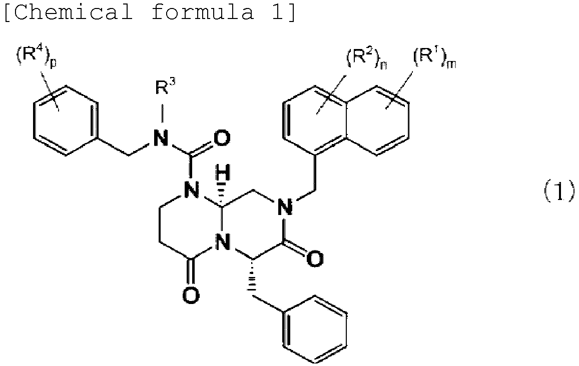

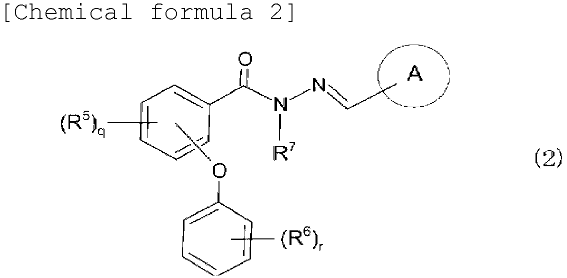

(In the formulas,
R1, R2, R4, R5, and R6 are the same or different from each other and are H, a halogen, nitro, cyano, OH, optionally substituted C1-C6 alkyl, optionally substituted C2-C6 alkenyl, optionally substituted C1-C6 alkoxy, aryl, or heteroaryl;
R3 and R7 are H, an optionally substituted C1-6 alkyl, or optionally substituted C2-6 alkenyl;
ring A is an optionally substituted aryl or optionally substituted heteroaryl;
m and q are any integer of 1-4;
n is any integer of 1-3;
p and r are any integer of 1-5.
However, excluding N-[(5-methyl-2-furyl)methylideneamino]-2-phenoxy-benzamide.)

Differentiation inducers containing one or more compounds selected from the compound group shown by formula (1) and formula (2), salts thereof, or solvates of these are provided as another example of suitable Wnt/β-catenin signal inhibitory compounds. In addition, differentiation inducers containing compounds shown by formula (8), salts thereof, or solvates of these are provided.

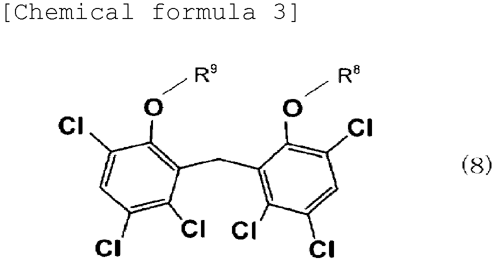

(In the formula,
R8 and R9 are the same or different from each other and are an optionally substituted C1-C6 alkyl or optionally substituted C2-C6 alkenyl.)

The above compounds are examples. Such Wnt/β-catenin signal inhibitory compounds are contemplated in the present disclosure as long as the compounds are derivatives of the above compounds and have a Wnt/β-catenin signal inhibitory effect. Whether or not a compound has a Wnt/β-catenin signal inhibitory effect can be determined by various means. For example, the luciferase activity in the cultured cells can serve as a standard.

### <Suspension culture>

In the present disclosure, the cell sheet recovered from the temperature-responsive culture dish as described above is suspension cultured. The suspension culture time can be selected as is appropriate within the range where the MMP activity in the shrunken cell sheet is sufficiently high and there is no problem with the stability of the cell sheet. As one example, a time between 8 hours and 24 hours can be selected as is appropriate. Suspension culture in the present disclosure means that the recovered cells are allowed to stand in a state suspended in culture broth without again being adhered to the container. The culture broth used in this step is preferably the culture broth used in the sheetmaking step. The cell sheet subjected to suspension culture maintains the extracellular matrix in the same way as the cell sheet immediately after sheetmaking and can be engrafted to another cell sheet, a substrate, or a disease target tissue.

The present inventors discovered that the MMP activity is increased significantly in the cell sheet by suspension culturing the cell sheet obtained during cell sheet production, and arrived at the present invention. As explained in the present disclosure, the MMP activity tended to increase both when a cell sheet was produced from bone marrow mononuclear cells and when exposed to a compound having a Wnt/β-catenin signal inhibitory action in the cell sheetmaking step, but cell sheets produced by exposure to a Wnt/β-catenin signal inhibitory compound had higher MMP activity than cell sheets produced from bone marrow mononuclear cells.

### <Measurement of fibrosis inhibitory effect>

### <MMP activity>

The present inventors reported that activation of hepatic stellate cells was suppressed in chronic liver damage model mice using liver cell sheets differentiated by the IC-2 compound (Patent Reference 1). Based on this, the present inventors focused on the matrix metalloprotease (MMP) group associated with decomposition of type I collagen, which serves as a major component of liver fibrosis inhibition, and discovered and reported that MMP-1 and MMP-14 are strongly involved in fibrosis in chronic and acute liver disease (Liver Forum, March 2017).

In the present disclosure, the MMP activity can be determined by measuring the enzyme activity using a fluorescence resonance energy transfer (FRET) peptide that generates fluorescence upon cleavage by MMP.

### <Measurement of hydroxyproline>

A frozen liver tissue fragment (cell sheet) is disrupted by a homogenizer, and a homogenate is obtained. This homogenate is frozen by nitrogen, thawed at room temperature, and then sonicated using an ultrasonic cell disrupter BioRuptur (Cosmo Bio, Tokyo, Japan). An equal amount of 12N concentrated hydrochloric acid is added to this disrupted solution to perform hydrolysis. The remaining homogenate is used in measurement of the amount of protein in the solution. After hydrolysis, and after cooling to room temperature, the hydrolysate is finely crushed by pipetting and centrifuged for five minutes at 3000 rpm at room temperature. The supernatant is placed in a 1.5 mL tube, and the hydrochloric acid is removed by a cold evaporator (Sakuma Seisakusho, Tokyo, Japan). Hydroxyproline is measured using a hydroxyproline quantification kit (BioVision, California, USA).

The amount of protein in the disrupted solution is measured by the Bradford method using a protein assay concentrated dye reagent (Bio-Rad, California, USA).

### <Histology studies>

Histology studies of the inhibitory effect on fibrosis are conducted using Sirius red stain and azan stain.

### EXAMPLES

The present invention is described further below through examples, but the present invention is not limited to these examples.

### Example 1

### MMP activity of trypsin-treated cell suspension and cell sheet, and synergistic effect by number of seeded cells

Bone marrow-derived mesenchymal stem cells (UE7T-13 cell line) were suspended in DMED/10% FBS and seeded to make respective seeded cell densities of 3.6 × 10⁴ cells/cm², 5.4 × 10⁴ cells/cm², and 7.2 × 10⁴ cells/cm² in temperature-responsive culture dishes and ordinary cell culture dishes. After four days, the temperature-responsive culture dishes were moved into a 32°C, 5% CO₂ incubator, and sheets were made. The cell sheets obtained were washed three times with PBS, then homogenized in the buffer of an MMP activity measurement kit (SensoLyte^{®} 520 MMP-1 Assay Kit Fluorimetric, (ANASPEC Co.: Cat#: AS-71150) and SensoLyte^{®} 520 MMP-14 Assay Kit Fluorimetric (ANASPEC Co.: Cat# AS72025)). The cells seeded in the ordinary cell culture dishes were dispersed in 0.025% trypsin/0.1 mM EDTA, recovered by centrifugation, then washed three times with PBS and homogenized in the buffer for the MMP activity measurement. The results of the measured MMP activity are shown in Table 1 and FIGS. 1A and 1B. The results in FIG. 1 show the results on MMP-1 (FIG. 1A) and MMP-14 (FIG. 1B) per cell. It was revealed that, when cultured at increased seeding densities, MMP-1 and MMP-14 become higher in cell sheets subjected to the suspension culture after making the sheet than in the trypsin-treated cell suspension. Also, it was revealed that the MMP-1 activity per cell increased when the cell sheet of the present disclosure was made by increasing the cell seeding density. It was a surprising result that the MMP-1 activity per cell was increased by increasing the seeding density. In addition, while the activity increased about four-fold in the trypsin-treated cell suspension when the seeding density was increased four-fold, the activity increased about five- to six-fold in the cell sheet of the present disclosure. Cell sheets of high cell seeding density also had excellent physical strength.

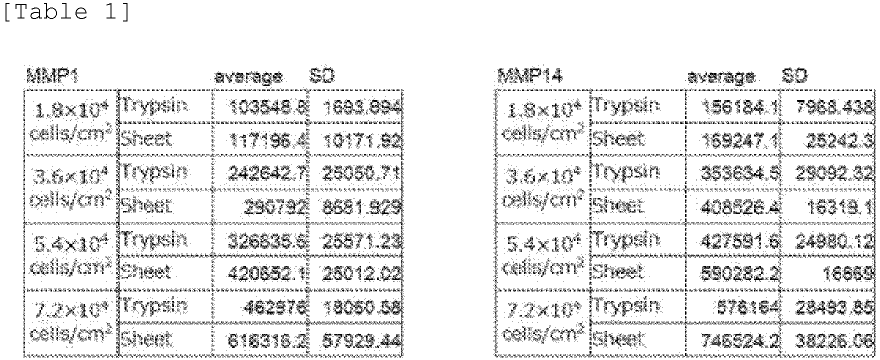

### Example 2

Adherent bone marrow mononuclear cells (Lonza Japan Ltd.) were suspended in DMED/20% FBS and seeded to make seeded cell densities of 1.8 × 10⁴ cells/cm², 3.6 × 10⁴ cells/cm², and 5.4 × 10⁴ cells/cm² in temperature-responsive culture dishes. On the next day of seeding, the medium was changed to DMEM/10% FBS containing 30 µM of IC-2. An embodiment using medium with DMSO added instead of the IC-2 compound was also implemented. Four days after adding IC-2 and DMSO, the respective media were again changed for a medium containing 30 µM of IC-2 or medium containing DMSO. After seven days, the temperature-responsive culture dishes were moved into a 20°C, 5% CO₂ incubator, and sheets were made. The cells that had been made into sheets were suspension cultured by being left to stand overnight at 20°C with 5% CO₂ in Petri dishes containing the same culture broth. After suspension culture, the cell sheets were washed three times with PBS, then homogenized in the buffer of an MMP activity measurement kit (SensoLyte^{®} 520 MMP-1 Assay Kit Fluorimetric, (ANASPEC Co.: Cat#: AS-71150) and SensoLyte^{®} 520 MMP-14 Assay Kit Fluorimetric (ANASPEC Co.: Cat#: AS-72025)). MMP-1 and MMP-14 were each measured at a fixed amount of protein. The MMP-1 and MMP-14 activity increased in both the DMSO- and IC-2-treated cell sheets when suspension cultured (not shown in drawing). The results on MMP-1 are shown in FIG. 2. These results revealed that the activity relative to protein is also increased by increasing the seeded density. Also, this tendency was more evident in cell sheets treated with IC-2 than with DMSO.

### Example 3

Bone marrow-derived mesenchymal stem cells (UE7T-13 cell line) were suspended in DMED/10% FBS and seeded to make a seeded cell density of 3.6 × 10⁴ cells/cm² in temperature-responsive culture dishes. On the next day of seeding, the medium was changed to DMEM/10% FBS containing 15 µM of IC-2. The same approach was also followed using medium with DMSO added instead of the IC-2 compound. Four days after seeding, the medium was again changed for a medium containing 15 µM of IC-2 or medium containing DMSO. After eight days, the temperature-responsive culture dishes were moved into a 20°C, 5% CO₂ incubator, and sheets were made. The cell sheets obtained were washed three times with PBS, then homogenized in the buffer of an MMP activity measurement kit (SensoLyte^{®} 520 MMP-1 Assay Kit Fluorimetric, (ANASPEC Co.: Cat#: AS-71150) and SensoLyte^{®} 520 MMP-14 Assay Kit Fluorimetric (ANASPEC Co.: Cat#: AS-72025)), and the MMP-1 and MMP-14 activities were measured. The MMP-1 and MMP-14 activity of cells prior to IC-2 and DMSO treatment was measured as day 0. Furthermore, the cells were not in the form of a sheet on day 0. The results on the MMP activity measured at a fixed amount of protein are shown in FIG. 3.

FIG. 3 revealed that the MMP-1 and MMP-14 activity in the cells is increased several fold by making the cells into sheets and that the IC-2 compound increases the activity more than DMSO.

## Claims

1. A cell sheet comprising bone marrow mononuclear cells, wherein the cell sheet is obtained by making bone marrow mononuclear cells from a bone marrow mononuclear cell suspension into a sheet, shrinking the sheet, and then suspension culturing the sheet for at least 8 hours, wherein the sheet has a high fibrosis inhibitory effect.

2. The cell sheet of Claim 1, wherein the bone marrow mononuclear cells are adherent bone marrow mononuclear cells.

3. The cell sheet of Claim 1, wherein the bone marrow mononuclear cells are mesenchymal stem cells.

4. The cell sheet of Claim 1, wherein the bone marrow mononuclear cells, when being made into a sheet, are exposed to a compound having a Wnt/β-catenin signal inhibitory action.

5. The cell sheet of Claim 4, wherein the compound having a Wnt/β-catenin signal inhibitory action is IC-2.

6. The cell sheet of Claim 1, which shows a higher fibrosis inhibitory effect.

7. The cell sheet of Claim 1, wherein the cell sheet obtained is applicable to various tissues.

8. The cell sheet of Claim 1, for use in the treatment of liver disease.

9. The cell sheet of Claim 1, which shows a higher matrix metalloprotease (MMP) activity.

10. A laminated cell sheet for use in the treatment of fibrosis obtained by laminating cell sheets of Claim 1.

11. An in vitro method for producing a cell sheet having a fibrosis inhibitory action comprising
a step for obtaining bone marrow mononuclear cells,
a step for suspending the bone marrow mononuclear cells in culture broth,
a step for seeding the suspension of bone marrow mononuclear cells onto a temperature-responsive culture dish and incubating,
a step for confirming that the cultured bone marrow mononuclear cells have become confluent, moving the temperature-responsive culture dish into a 20°C environment, and obtaining a cell sheet,
and a step for suspension culturing the cell sheet obtained for at least 8 hours.

12. The method for producing a cell sheet of Claim 11, wherein the bone marrow mononuclear cells are adherent bone marrow mononuclear cells.

13. The method for producing a cell sheet of Claim 11, wherein the bone marrow mononuclear cells are mesenchymal stem cells.

14. The method for producing a cell sheet of Claim 11, wherein the culture broth contains a compound having a Wnt/β-catenin signal inhibitory action in the step for seeding the suspension of bone marrow mononuclear cells onto a temperature-responsive culture dish and incubating.

15. The method for producing a cell sheet of Claim 14, wherein the compound having a Wnt/β-catenin signal inhibitory action is IC-2.

16. The method for producing a cell sheet of Claim 11, wherein the cell sheet obtained is applicable to various tissues.

17. The method for producing a cell sheet of Claim 11, wherein the cell sheet obtained is a cell sheet for treating liver disease.

## Patentansprüche

1. Zellschicht, die mononukleäre Knochenmarkzellen umfasst, wobei die Zellschicht durch Verarbeiten mononukleärer Knochenmarkzellen aus einer Suspension mononukleärer Knochenmarkzellen zu einer Schicht, Schrumpfen der Schicht und anschließendes Kultivieren der Schicht mittels Suspensionskultur für wenigstens 8 Stunden erhalten wird, wobei die Schicht eine starke Fibrose-inhibierende Wirkung aufweist.

2. Zellschicht nach Anspruch 1, wobei die mononukleären Knochenmarkzellen adhärente mononukleäre Knochenmarkzellen sind.

3. Zellschicht nach Anspruch 1, wobei die mononukleären Knochenmarkzellen mesenchymale Stammzellen sind.

4. Zellschicht nach Anspruch 1, wobei die mononukleären Knochenmarkzellen bei der Verarbeitung zu einer Schicht einer Verbindung mit inhibierender Wirkung auf das Wnt/β-Catenin-Signal ausgesetzt werden.

5. Zellschicht nach Anspruch 4, wobei die Verbindung mit inhibierender Wirkung auf das Wnt/β-Catenin-Signal IC-2 ist.

6. Zellschicht nach Anspruch 1, die eine stärkere Fibrose-inhibierende Wirkung aufweist.

7. Zellschicht nach Anspruch 1, wobei die erhaltene Zellschicht für verschiedene Gewebe anwendbar ist.

8. Zellschicht nach Anspruch 1 zur Verwendung bei der Behandlung von Lebererkrankungen.

9. Zellschicht nach Anspruch 1, die eine stärkere Wirkung von Matrix-Metalloproteasen (MMP) aufweist.

10. Laminierte Zellschicht zur Verwendung bei der Behandlung von Fibrose, die durch Laminieren von Zellschichten nach Anspruch 1 erhalten wird.

11. In-Vitro-Verfahren zur Herstellung einer Zellschicht mit Fibrose-inhibierender Wirkung, umfassend
einen Schritt zum Erhalten mononukleärer Knochenmarkzellen,
einen Schritt zum Suspendieren der mononukleären Knochenmarkzellen in Kulturlösung,
einen Schritt zum Aufbringen der Suspension mononukleärer Knochenmarkzellen auf eine thermoresponsive Kulturschale und Inkubieren,
einen Schritt zum Bestätigen, dass die kultivierten mononukleären Knochenmarkzellen konfluent geworden sind, Verbringen der thermoresponsiven Kulturschale in eine Umgebung von 20 °C und Erhalten einer Zellschicht,
und einen Schritt zum Kultivieren der erhaltenen Zellschicht mittels Suspensionskultur für wenigstens 8 Stunden.

12. Verfahren zur Herstellung einer Zellschicht nach Anspruch 11, wobei die mononukleären Knochenmarkzellen adhärente mononukleäre Knochenmarkzellen sind.

13. Verfahren zur Herstellung einer Zellschicht nach Anspruch 11, wobei die mononukleären Knochenmarkzellen mesenchymale Stammzellen sind.

14. Verfahren zur Herstellung einer Zellschicht nach Anspruch 11, wobei in dem Schritt zum Aufbringen der Suspension mononukleärer Knochenmarkzellen auf eine thermoresponsive Kulturschale und Inkubieren die Kulturlösung eine Verbindung mit inhibierender Wirkung auf das Wnt/β-Catenin-Signal enthält.

15. Verfahren zur Herstellung einer Zellschicht nach Anspruch 14, wobei die Verbindung mit inhibierender Wirkung auf das Wnt/β-Catenin-Signal IC-2 ist.

16. Verfahren zur Herstellung einer Zellschicht nach Anspruch 11, wobei die erhaltene Zellschicht für verschiedene Gewebe anwendbar ist.

17. Verfahren zur Herstellung einer Zellschicht nach Anspruch 11, wobei die erhaltene Zellschicht eine Zellschicht zur Behandlung von Lebererkrankungen ist.

## Revendications

1. Feuille cellulaire comprenant des cellules mononucléaires de moelle osseuse, ladite feuille cellulaire étant obtenue par formation en feuille des cellules mononucléaires de moelle osseuse d'une suspension de cellules mononucléaires de moelle osseuse, rétraction de la feuille, puis par culture en suspension de la feuille pendant au moins 8 heures, ladite feuille ayant un effet inhibiteur élevé sur la fibrose.

2. Feuille cellulaire selon la revendication 1, où les cellules mononucléaires de moelle osseuse sont des cellules mononucléaires de moelle osseuse adhérentes.

3. Feuille cellulaire selon la revendication 1, où les cellules mononucléaires de moelle osseuse sont des cellules souches mésenchymateuses.

4. Feuille cellulaire selon la revendication 1, où les cellules mononucléaires de moelle osseuse, lorsqu'elles sont formées en feuille, sont exposées à un composé ayant une action inhibitrice de la signalisation Wnt/β-caténine.

5. Feuille cellulaire selon la revendication 4, où le composé ayant une action inhibitrice de la signalisation Wnt/β-caténine est l'IC-2.

6. Feuille cellulaire selon la revendication 1, présentant un effet inhibiteur supérieur sur la fibrose.

7. Feuille cellulaire selon la revendication 1, où la feuille cellulaire obtenue est applicable à différents tissus.

8. Feuille cellulaire selon la revendication 1, destinée à être utilisée pour le traitement de maladies hépatiques.

9. Feuille cellulaire selon la revendication 1, présentant une activité supérieure sur la métalloprotéase matricielle (MMP).

10. Feuille cellulaire stratifiée, destinée à être utilisée pour le traitement d'une fibrose, obtenue par stratification de feuilles cellulaires selon la revendication 1.

11. Procédé de production in vitro d'une feuille cellulaire ayant un effet inhibiteur sur la fibrose, comprenant
une étape d'obtention de cellules mononucléaires de moelle osseuse,
une étape de suspension des cellules mononucléaires de moelle osseuse dans un bouillon de culture,
une étape d'ensemencement de la suspension de cellules mononucléaires de moelle osseuse sur un récipient de culture sensible à la température et d'incubation,
une étape de confirmation que les cellules mononucléaires de moelle osseuse cultivées sont devenues confluentes, de déplacement du récipient de culture sensible à la température vers un environnement à 20 °C, et d'obtention d'une feuille cellulaire,
et une étape de culture en suspension de la feuille cellulaire obtenue pendant au moins 8 heures.

12. Procédé de production d'une feuille cellulaire selon la revendication 11, où les cellules mononucléaires de moelle osseuse sont des cellules mononucléaires de moelle osseuse adhérentes.

13. Procédé de production d'une feuille cellulaire selon la revendication 11,
où les cellules mononucléaires de moelle osseuse sont des cellules souches mésenchymateuses.

14. Procédé de production d'une feuille cellulaire selon la revendication 11, où le bouillon de culture contient un composé ayant une action inhibitrice de la signalisation Wnt/β-caténine lors de l'étape d'ensemencement de la suspension de cellules mononucléaires de moelle osseuse sur un récipient de culture sensible à la température et d'incubation.

15. Procédé de production d'une feuille cellulaire selon la revendication 14, où le composé ayant une action inhibitrice de la signalisation Wnt/β-caténine est l'IC-2.

16. Procédé de production d'une feuille cellulaire selon la revendication 11, où la feuille cellulaire obtenue est applicable à différents tissus.

17. Procédé de production d'une feuille cellulaire selon la revendication 11, où la feuille cellulaire obtenue est une feuille cellulaire pour le traitement de maladies hépatiques.
